# EUROPEAN PATENT SPECIFICATION

(11) **EP 2 725 011 B1**
(45) Date of publication and mention of the grant of the patent: **27.10.2021**
(21) Application number: 12801908.0
(22) Date of filing: 15.06.2012
(51) Int. Cl.: G03F 7/004, G03F 7/085, G03F 7/027, G03F 7/038, G03F 7/031, C07C 231/02, C07C 235/34, C07C 235/38

(54) **NOVEL BASE GENERATOR AND ADHESION ENHANCER**
NEUER BASENGENERATOR UND HAFTVERSTÄRKER
NOUVEAU GÉNÉRATEUR DE BASE ET AMPLIFICATEUR DE L'ADHÉSION

(30) Priority: 24.06.2011 JP 2011141229
(43) Date of publication of application: 30.04.2014
(73) Proprietor: Tokyo Ohka Kogyo Co., Ltd., Kawasaki-shi, Kanagawa 211-0012 (JP); Daicel Corporation, Osaka-shi, Osaka 530-0011 (JP)
(72) Inventor: SHIOTA, Dai, Kawasaki-shi Kanagawa 211-0012 (JP); KUROKO, Mayumi, Kawasaki-shi Kanagawa 211-0012 (JP); NODA, Kunihiro, Kawasaki-shi Kanagawa 211-0012 (JP); TADOKORO, Yoshinori, Kawasaki-shi Kanagawa 211-0012 (JP); AKAI, Yasuyuki, Himeji-shi Hyogo 671-1283 (JP); TAKAI, Hideyuki, Himeji-shi Hyogo 671-1283 (JP)
(74) Representative: Plasseraud IP
(86) International application number: PCT/JP2012/065306
(87) International publication number: WO 2012/176693

(56) References cited:
- EP-A1- 2 179 984
- WO-A1-2008/003141
- WO-A1-2010/113813
- WO-A2-2009/091225
- CN-A- 101 591 310
- JP-A- 1 246 271
- JP-A- H08 127 572
- JP-A- 2005 517 737
- JP-A- 2011 052 214
- JP-A- 2011 095 635
- US-A- 4 931 471
- HAJRA, SAUMEN ET AL.: 'Lewis acid catalyzed intramolecular halo-arylation of tethered alkenes using N-halosuccinimide (NXS) as the halogen source: a general method for the synthesis of chromanones, chromans, quinolones, tetrahydroquinolines, and tetralins' TETRAHEDRON LETTERS vol. 46, no. 49, 2005, pages 8599 - 8603, XP005149433
- ZHOU, PENG ET AL.: 'Microscale CD method for determining absolute configurations of acyclic amino tetrols and amino pentols. Structures of aminobacteriohopanepolyols from the methylotrophic bacterium Methylococcus luteus' JOURNAL OF THE AMERICAN CHEMICAL SOCIETY vol. 113, no. 10, 1991, pages 4040 - 4042, XP055140716
- PAGANI, G. ET AL.: 'Cinnamic acid derivatives with phytotoxic activity.' II, FARMACO, EDIZIONE SCIENTIFICA vol. 28, no. 3, 1973, pages 231 - 242, XP008172726
- None

## Description

### TECHNICAL FIELD

The present invention relates to a novel base generator and adhesion enhancer which is suitably used for a negative-type photosensitive resin composition.

### BACKGROUND ART

A negative-type photosensitive resin composition has a property of being cured when irradiated with an electromagnetic wave such as an ultraviolet ray. Since it is possible to obtain a pattern having a desired shape by curing a part of the negative-type photosensitive resin composition through irradiation of the part with light, the negative-type photosensitive resin composition has been widely used for various usages such as display devices, semiconductor devices, electronic component parts, and micro-electromechanical systems (MEMS). For example, in the display device, it is used as a material for a flattened film, an insulating film, a color filter, a black matrix, a spacer, a partition of a liquid crystal display, an organic EL display.

In order to ensure reliability of the products, the negative-type photosensitive resin composition is required to have high adhesiveness which enables attaining close contact with a substrate in the case of forming a minute pattern. Accordingly, a negative-type photosensitive resin composition which contains an amine-based silane coupling agent as an adhesion enhancer has been proposed (see Patent Document 1).
[Patent Document 1] Japanese Unexamined Patent Application, Publication No.2000-035670
[Patent Document 2] Japanese Unexamined Patent Application, Publication No.2011-052214
Other background art is described in WO 2010/113813, US 4931471, JP H08-127572, CN 101591310, WO 2008/003141, EP 2179984 and WO 2009/091225.

### DISCLOSURE OF THE INVENTION

### Problems to be Solved by the Invention

By the way, from the viewpoint of further improvements in productivity, there has recently been a demand for a negative-type photosensitive resin composition which is capable of forming a pattern having a favorable shape at a low light exposure.

However, in the case where the amine-based silane coupling agent is contained as an adhesion enhancer as in Patent Document 1, there has been a problem that it is necessary to increase light exposure for pattern formation despite the improvement in adhesiveness to the substrate.

The present invention was accomplished in view of the above-described problem, and an object thereof is to provide a novel compound suitable for obtaining a negative-type photosensitive resin composition capable of forming a pattern having favorable adhesiveness at a low light exposure.

### Means for Solving the Problems

The inventors had conducted extensive researches to attain the above-described object. As a result, they had found that it is possible to solve the problem by incorporating a specific compound into a negative-type photosensitive resin composition and thus accomplished the present invention.
The invention provides a use of a compound as a base generator or adhesion enhancer as described herebelow and in the appended claims.

More specifically, the compound used as a base generator or adhesion enhancer according to the present invention is represented by the following formula (1): 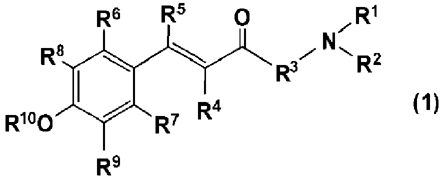 (wherein R¹ and R² each independently indicate a hydrogen atom or an organic group,
provided that at least one of R¹ and R² indicates an organic group;
R¹ and R² may be bonded to form a ring structure and may contain a hetero atom bond;
R³ indicates a single bond or an organic group;
R⁴ and R⁵ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group;
R⁶ and R⁷ each independently indicate a hydrogen atom, a halogen atom, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group,
R⁸ and R⁹ each independently indicate a a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group, or an organic group;
two or more of R⁶, R⁷, R⁸, and R⁹ may be bonded to form a ring structure and may contain a hetero atom bond; and
R¹⁰ indicates an alkyl group having 1 to 12 carbons).

As a compound having a structure similar to that of the compound represented by the above formula (1), a base generator represented by the following formula is disclosed in Patent Document 2. The base generator is cyclized through electromagnetic wave irradiation and heating to form a base (NHR²¹R²²). 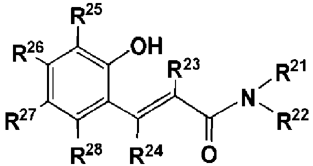 (wherein R²¹ and R²² each independently are a hydrogen atom or an organic group and may be the same or different;
R²¹ and R²² may be bonded to form a ring structure and may contain a hetero atom bond,
provided that at least one of R²¹ and R²² is an organic group;
R²³ and R²⁴ each independently are a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group and may be the same or different,
provided that at least one of R²³ and R²⁴ is a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group;
R²⁵, R²⁶, R²⁷, and R²⁸ each are a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group, or an organic group and may be the same or different; and
two or more of R²⁵, R²⁶, R²⁷, and R²⁸ may be bonded to form a ring structure and may contain a hetero atom bond.)

However, as a result of the inventors' confirmation, a favorable micropatterning property was not attained by incorporating the base generator described in Patent Document 2 into a negative-type photosensitive resin composition due to consumption of optical energy by the cyclization.

In contrast, the compound represented by the above formula (1) is free from the cyclization reaction since R⁶ and R⁷ are never hydroxyl groups and is capable of giving a favorable micropatterning property.

Since the compound represented by the above formula (1) generates a base by electromagnetic wave irradiation or heating, the compound functions also as a base generator. Further, since the compound represented by the above formula (1) attains the effect of enhancing adhesion to a substrate when incorporated into, for example, a negative-type photosensitive resin composition, the compound functions also as an adhesion enhancer.

### Effects of the Invention

It is possible to obtain a negative-type photosensitive resin composition which is capable of forming a pattern having favorable adhesiveness at a low light exposure by incorporating the compound according to the present invention into a negative-type photosensitive resin composition.

### PREFERRED MODE FOR CARRYING OUT THE INVENTION

The compound according to the present invention is represented by the following formula (1).

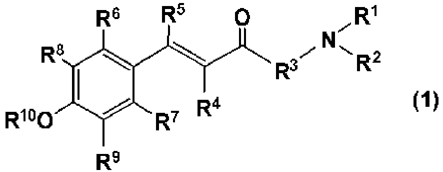

In the formula (1), R¹ and R² each independently indicate a hydrogen atom or an organic group, but at least one of R¹ and R² indicates an organic group.

Examples of the organic group in R¹ and R² include an alkyl group, an alkenyl group, a cycloalkyl group, a cycloalkenyl group, an aryl group, an aralkyl group. The organic group may contain in the organic group a bond or a substituent other than those of a hydrocarbon group, such as those of a hetero atom. Also, the organic group may be any one of straight chain, branched chain, and cyclic organic groups.

The organic group ordinarily is monovalent but can be a polyvalent of which a valence is 2 or more in the case of forming the ring structure.

R¹ and R² may be bonded to form a ring structure and may further contain a hetero atom bond. Examples of the ring structure include a heterocycloalkyl group, a heteroaryl group, and the ring structure may be a condensed ring.

The bond other than hydrocarbon group bond in the organic group of R¹ and R² is not particularly limited insofar as the effects of the present invention are not impaired, and examples thereof include a bond including a hetero atom such as an oxygen atom, a nitrogen atom, and a silicon atom. Specific examples thereof include an ether bond, a thioether bond, a carbonyl bond, a thiocarbonyl bond, an ester bond, an amide bond, a urethane bond, an imino bond (-N=C(-R)-, - C(=NR)-; R indicates a hydrogen atom or an organic group), a carbonate bond, a sulfonyl bond, a sulfinyl bond, and an azo bond.

From the viewpoint of heat resistance, preferred examples of the bond other than hydrocarbon group bond in the organic group of R¹ and R² include an ether bond, a thioether bond, a carbonyl bond, a thiocarbonyl bond, an ester bond, an amide bond, a urethane bond, an imino bond (-N=C(-R)-, -C(=NR)-; R indicates a hydrogen atom or a monovalent organic group), a carbonate bond, a sulfonyl bond, and a sulfinyl bond.

The substituent other than hydrocarbon group in the organic group of R¹ and R² is not particularly limited insofar as the effects of the present invention are not impaired, and examples thereof include a halogen atom, a hydroxyl group, a mercapto group, a sulfide group, a cyano group, an isocyano group, a cyanato group, an isocyanato group, a thiocyanato group, an isothiocyanato group, a silyl group, a silanol group, an alkoxy group, an alkoxycarbonyl group, a carbamoyl group, a thiocarbamoyl group, a nitro group, a nitroso group, a carboxyl group, a carboxylate group, an acyl group, an acyloxy group, a sulfino group, a sulfo group, a sulfonato group, a phosphino group, a phosphinyl group, a phosphono group, a phosphonato group, a hydroxyimino group, an alkylether group, an alkenylether group, an alkylthioether group, an alkenylthioether group, an arylether group, an arylthioether group, an amino group (-NH₂, -NHR, -NRR'; R and R' each independently indicate a hydrocarbon group). The hydrogen atom contained in the substituent may be substituted with a hydrocarbon group. Also, the hydrocarbon group contained in the substituent may be any one of straight chain, branched chain, and cyclic hydrocarbon groups.

Preferred examples of the substituent other than hydrocarbon group in the organic group of R¹ and R² include a halogen atom, a hydroxyl group, a mercapto group, a sulfide group, a cyano group, an isocyano group, a cyanato group, isocyanato group, a thiocyanato group, an isothiocyanato group, a silyl group, a silanol group, an alkoxy group, an alkoxycarbonyl group, a carbamoyl group, a thiocarbamoyl group, a nitro group, a nitroso group, a carboxyl group, a carboxylate group, an acyl group, an acyloxy group, a sulfino group, a sulfo group, a sulfonato group, a phosphino group, a phosphinyl group, a phosphono group, a phosphonato group, a hydroxyimino group, an alkylether group, an alkenylether group, an alkylthioether group, an alkenylthioether group, an arylether group, and an arylthioether group.

Among the above, as R¹ and R², at least one of R¹ and R² is preferably an alkyl group having 1 to 12 carbon atoms or an aryl group having 1 to 12 carbon atoms, or R¹ and R² are preferably bonded to form a heterocycloalkyl group or a heteroaryl group having 2 to 20 carbon atoms. Examples of the heterocycloalkyl group include a piperidino group, a morpholino group, and examples of the heteroaryl group include an imidazolyl group, a pyrazolyl group.

In the formula (1), R³ indicates a single bond or an organic group.

Examples of the organic group in R³ include groups each of which is obtainable by removing a hydrogen atom from an alkyl group, an alkenyl group, a cycloalkyl group, a cycloalkenyl group, an aryl group, an aralkyl group. The organic group may contain a substituent in the organic group. Examples of the substituent include those exemplified in R¹ and R². Also, the organic group may be either one of straight chain or branched chain organic groups.

Among the above, R³ is preferably a single bond or a group obtainable by removing a hydrogen atom from an alkyl group having 1 to 12 carbon atoms or an aryl group having 1 to 12 carbon atoms.

In the formula (1), R⁴ and R⁵ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group.

Examples of the organic group in R⁴ and R⁵ include those exemplified in R¹ and R². The organic group may contain in the organic group a bond or a substituent other than those of a hydrocarbon group, such as those of a hetero atom as is the case with R¹ and R². Also, the organic group may be any one of straight chain, branched chain, and cyclic organic groups.

Among the above, R⁴ and R⁵ each independently are preferably a hydrogen atom, an alkyl group having 1 to 10 carbon atoms, a cycloalkyl group having 4 to 13 carbon atoms, a cycloalkenyl group having 4 to 13 carbon atoms, an aryloxyalkyl group having 7 to 16 carbon atoms, an aralkyl group having 7 to 20 carbon atoms, an alkyl group containing a cyano group and having 2 to 11 carbon atoms, an alkyl group containing a hydroxyl group and having 1 to 10 carbon atoms, an alkoxy group having 1 to 10 carbon atoms, an amide group having 2 to 11 carbon atoms, an alkylthiol group having 1 to 10 carbon atoms, an acyl group having 1 to 10 carbon atoms, an ester group having 2 to 11 carbon atoms (-COOR, -OCOR; R indicates a hydrocarbon group), an aryl group having 6 to 20 carbon atoms, an aryl group substituted with an electron donating group and/or an electron withdrawing group and having 6 to 20 carbon atoms, a benzyl group substituted with an electron donating group and/or an electron withdrawing group, a cyano group, or a methylthio group. More preferably, both of R⁴ and R⁵ are hydrogen atoms, or R⁴ is the methyl group and R⁵ is the hydrogen atom.

In the formula (1), R⁶, R⁷, R⁸, and R⁹ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group, or an organic group, provided that R⁶ and R⁷ are never hydroxyl groups.

Examples of the organic group in R⁶, R⁷, R⁸, and R⁹ include those exemplified in R¹ and R². The organic group may contain in the organic group a bond or a substituent other than those of a hydrocarbon group, such as those of a hetero atom as is the case with R¹ and R². Also, the organic group may be any one of straight chain, branched chain, and cyclic organic groups.

In the formula (1), R⁶ and R⁷ are never hydroxyl groups. In the case where R⁶ and R⁷ are hydroxyl groups, the cyclization reaction occurs due to electromagnetic wave irradiation and heating as is described in Patent Document 2. Therefore, it is impossible to attain a favorable micropatterning property by incorporating such a compound into a negative-type photosensitive resin composition due to consumption of optical energy by the cyclization. In contrast, since R⁶ and R⁷ are never hydroxyl groups, the compound represented by the formula (1) is free from the cyclization reaction and enables attaining a favorable micropatterning property when incorporated into a negative-type photosensitive resin composition.

Two or more of R⁶, R⁷, R⁸, and R⁹ may be bonded to form a ring structure and may contain a hetero atom bond. Examples of the ring structure include a heterocycloalkyl group, a heteroaryl group, and the ring structure may be a condensed ring. For example, two or more of R⁶, R⁷, R⁸, and R⁹ may be bonded to form a condensed ring such as naphthalene, anthracene, phenanthrene, and indene by sharing an atom of a benzene ring to which R⁶, R⁷, R⁸, and R⁹ are bonded.

Among the above, R⁶, R⁷, R⁸, and R⁹ each independently are preferably a hydrogen atom, an alkyl group having 1 to 10 carbon atoms, a cycloalkyl group having 4 to 13 carbon atoms, a cycloalkenyl group having 4 to 13 carbon atoms, an aryloxyalkyl group having 7 to 16 carbon atoms, an aralkyl group having 7 to 20 carbon atoms, an alkyl group containing a cyano group and having 2 to 11 carbon atoms, an alkyl group containing a hydroxyl group and having 1 to 10 carbon atoms, an alkoxy group having 1 to 10 carbon atoms, an amide group having 2 to 11 carbon atoms, an alkylthiol group having 1 to 10 carbon atoms, an acyl group having 1 to 10 carbon atoms, an ester group having 2 to 11 carbon atoms, an aryl group having 6 to 20 carbon atoms, an aryl group substituted with an electron donating group and/or an electron withdrawing group and having 6 to 20 carbon atoms, a benzyl group substituted with an electron donating group and/or an electron withdrawing group, a cyano group, a methylthio group, or a nitro group.

Also, as R⁶, R⁷, R⁸, and R⁹, it is preferable that two or more of R⁶, R⁷, R⁸, and R⁹ are bonded to form a condensed ring such as naphthalene, anthracene, phenanthrene, and indene by sharing an atom of a benzene ring to which R⁶, R⁷, R⁸, and R⁹ are bonded from the viewpoint of the absorption wavelength becoming a long wavelength.

More preferably, all of R⁶, R⁷, R⁸, and R⁹ are hydrogen atoms, or any one of R⁶, R⁷, R⁸, and R⁹ is the nitro group with the rest being hydrogen atoms.

In the formula (1), R¹⁰ indicates an alkyl group having 1 to 12 carbon atoms.

Among the above, R¹⁰ is preferably methyl group.

Among the compounds represented by the formula (1), particularly preferred examples include those represented by the following formulas.

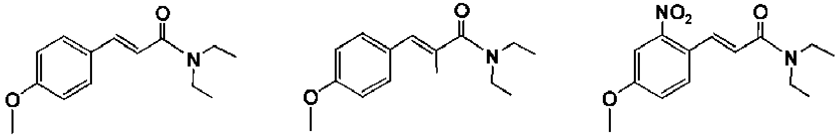

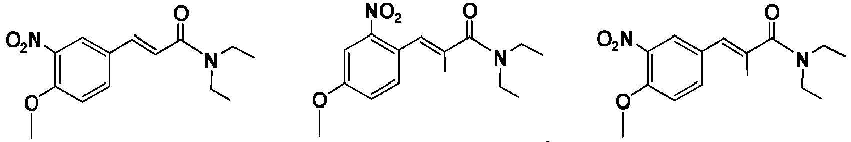

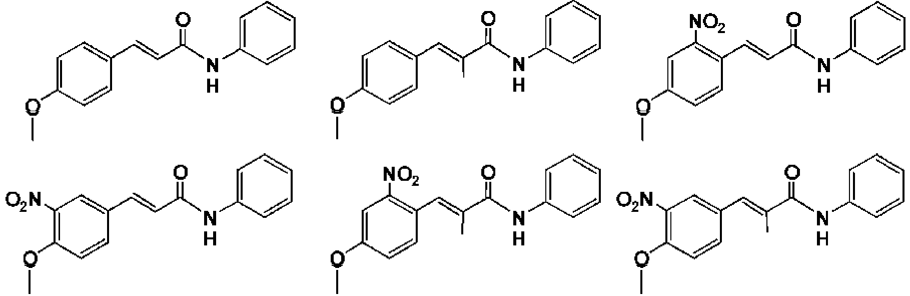

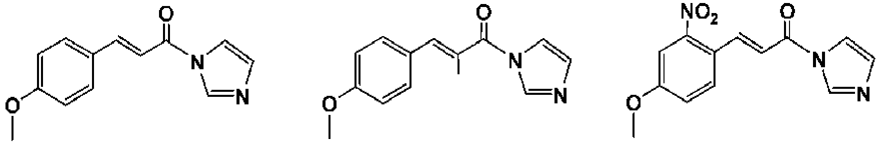

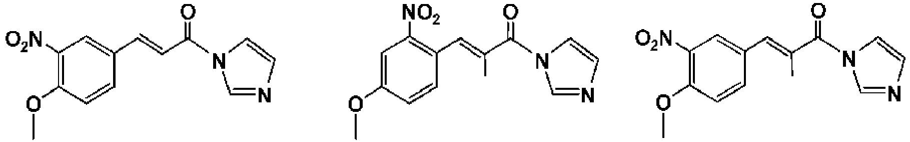

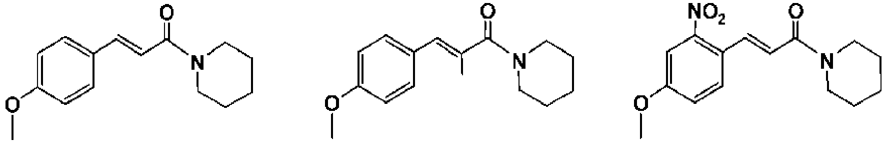

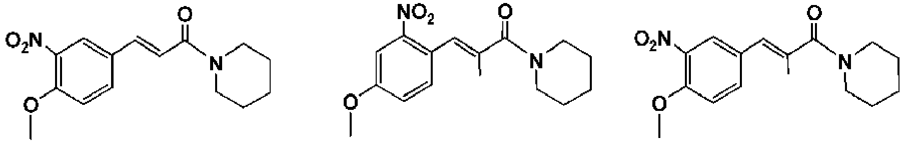

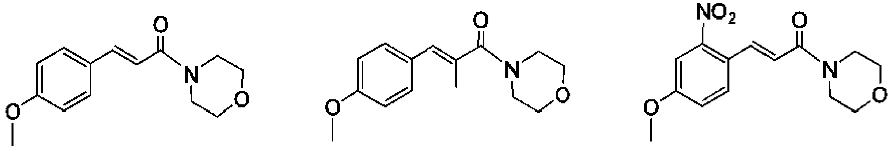

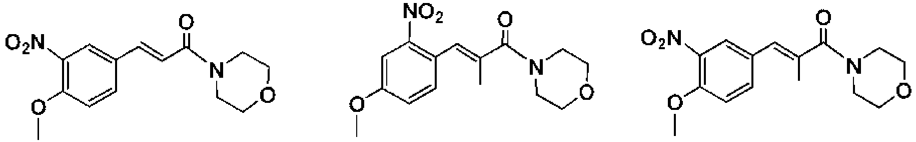

It is possible to synthesize the compounds represented by the formula (1) as described in Examples described later.

The compounds represented by the formula (1) have the favorable solubility into organic solvents and is capable of attaining a favorable micropatterning property when contained in the negative-type photosensitive resin composition. Examples of the negative-type photosensitive resin composition include those containing an alkali-soluble resin, a photopolymerizable monomer, a photopolymerization initiator, the compound represented by the formula (1), and an organic solvent. Hereinafter, the negative-type photosensitive resin composition will be described in detail.

As the alkali-soluble resin contained in the negative-type photosensitive resin composition, conventionally known alkali-soluble resins are usable without particular limitation. The alkali-soluble resin may be the one which has an ethylenic unsaturated group or the one which does not have any ethylenic unsaturated group.

In the present specification, the term "alkali-soluble resin" means that, in the case where a resin film having a film thickness of 1 µm is formed on a substrate by using a resin solution (solvent: propylene glycol monomethyl ether acetate) having a resin concentration of 20 mass%, a film thickness of 0.01 µm or more is dissolved when the substrate is immersed in a 2.38 mass% tetramethyl ammonium hydroxide (TMAH) aqueous solution for 1 minute.

As the alkali-soluble resin having ethylenic unsaturated group, for example, resins obtainable by causing a reaction of a reaction product of an epoxy compound and unsaturated carboxylic acid with a polybasic acid anhydride are usable.

Among them, the resin represented by the following formula (a-1) is preferred. The resin represented by the formula (a-1) is preferred since the resin itself has high photo-curability.

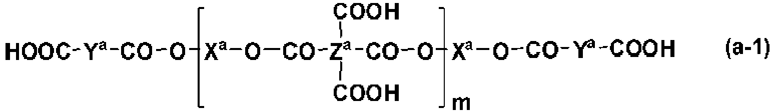

In the above formula (a-1), X^{a} indicates a group represented by the following formula (a-2).

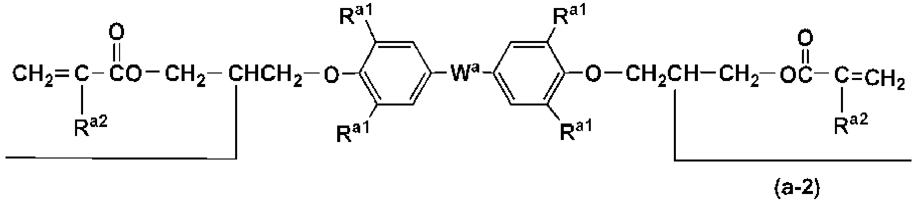

In the above formula (a-2), R^{a1} each independently indicates a hydrogen atom, a hydrocarbon group having 1 to 6 carbon atoms, or a halogen atom; R^{a2} each independently indicates a hydrogen atom or a methyl group; and W^{a} indicates a single bond or a group represented by the following formula (a-3) .

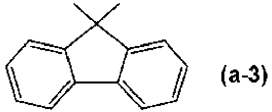

Also, in the formula (a-1), Y^{a} indicates a residue obtainable by removing an acid anhydride group (-CO-O-CO-) from dicarboxylic anhydride. Examples of the dicarboxylic anhydride include maleic anhydride, succinic anhydride, itaconic anhydride, phthalic anhydride, tetrahydrophthalic anhydride, hexahydrophthalic anhydride, methylendomethylenetetrahydrophthalic anhydride, chlorendic anhydride, methyltetrahydrophthalic anhydride, anhydrous glutaric acid.

In the formula (a-1), Z^{a} indicates a residue obtainable by removing 2 acid anhydride groups from tetracarboxylic acid dianhydride. Examples of the tetracarboxylic acid dianhydride include pyromellitic dianhydride, benzophenonetetracarboxylic dianhydride, biphenyltetracarboxylic dianhydride, biphenylethertetracarboxylic dianhydride.

In the formula (a-1), m indicates an integer of 0 to 20.

As the alkali-soluble resin having ethylenic unsaturated group, polyester(meth)acrylate obtainable by causing a reaction of a polyester prepolymer obtained by condensation of polyvalent alcohols with monobasic acid or polybasic acid with (meth)acrylic acid; polyurethane(meth)acrylate obtainable by causing a reaction of polyol with a compound having 2 isocyanate groups and then performing a reaction with (meth)acrylic acid; an epoxy(meth)acrylate resin obtainable by causing a reaction of an epoxy resin such as a bisphenol A-type epoxy resin, a bisphenol F-type epoxy resin, a bisphenol S-type epoxy resin, a phenol or cresol novolac-type epoxy resin, a resol-type epoxy resin, a triphenolmethane-type epoxy resin, polycarboxylic acid polyglycidyl ester, polyol polyglycidyl ester, an aliphatic or alicyclic epoxy resin, an amine epoxy resin, and a dihydroxybenzene-type epoxy resin with (meth)acrylic acid may be used.

In the present specification, the term "(meth)acrylic acid" means both of acrylic acid and methacrylic acid. Likewise, the term "(meth)acrylate" means both of acrylate and methacrylate.

As the alkali-soluble resin which does not have any ethylenic unsaturated group, a resin which is obtainable by copolymerizing at least an unsaturated carboxylic acid, an epoxy group-containing unsaturated compound which does not have any alicyclic group, and an alicyclic group-containing unsaturated compound may be used.

Examples of the unsaturated carboxylic acid include monocarboxylic acid such as (meth)acrylic acid and crotonic acid; dicarboxylic acid such as maleic acid, fumaric acid, citraconic acid, mesaconic acid, and itaconic acid; anhydrides of these dicarboxylic acids. Among these, (meth)acrylic acid and maleic anhydride are preferred from the viewpoints of copolymerization reactivity, alkali solubility of the obtained resin, easy availability, and so forth. These unsaturated carboxylic acids may be used alone or in combination of two or more kinds thereof.

Examples of the epoxy group-containing unsaturated compound which does not have any alicyclic group include (meth)acrylic acid epoxyalkyl esters such as glycidyl (meth)acrylate, 2-methylglycidyl (meth)acrylate, 3,4-epoxybutyl (meth)acrylate, 6,7-epoxyheptyl (meth)acrylate, 3,4-epoxycyclohexyl (meth)acrylate; α-alkylacrylic acid epoxyalkyl esters such as glycidyl α-ethylacrylate, glycidyl α-n-propylacrylate, glycidyl α-n-butylacrylate, and 6,7-epoxyheptyl α-ethylacrylate; glycidyl ethers such as o-vinylbenzyl glycidyl ether, m-vinylbenzyl glycidyl ether, and p-vinylbenzyl glycidyl ether. Among these, glycidyl (meth)acrylate, 2-methylglycidyl (meth)acrylate, 6,7-epoxyheptyl (meth)acrylate, o-vinylbenzyl glycidyl ether, m-vinylbenzyl glycidyl ether and p-vinylbenzyl glycidyl ether are preferred from the viewpoints of copolymer reactivity, resin strength after curing and so forth. These epoxy group-containing unsaturated compounds may be used alone or in combination of two or more kinds thereof.

As the alicyclic group-containing unsaturated compound, an unsaturated compound may be used without particular limitation insofar as the unsaturated compound has an alicyclic group. The alicyclic group may be monocyclic or polycyclic. Examples of monocyclic alicyclic group include a cyclopentyl group, a cyclohexyl group. Examples of the polycyclic alicyclic group include an adamantyl group, a norbornyl group, an isobornyl group, a tricyclononyl group, a tricyclodecyl group, a tetracyclododecyl group. More specifically, examples of the alicyclic group-containing unsaturated compound include the compounds represented by the following formula.

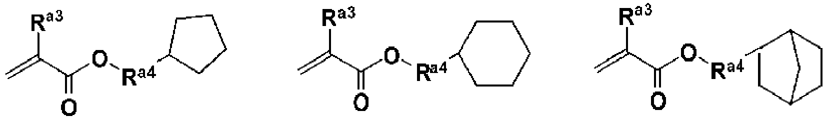

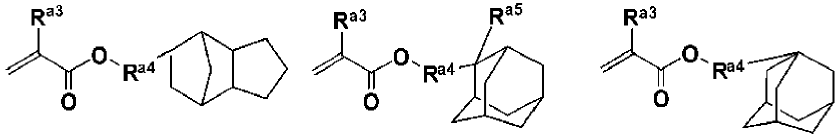

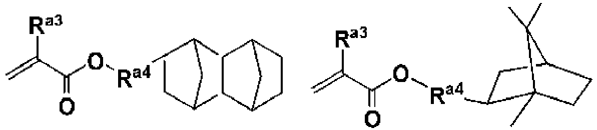

In the above formula, R^{a3} indicates a hydrogen atom or a methyl group; R^{a4} indicates a single bond or a divalent aliphatic saturated hydrocarbon group having 1 to 6 carbon atoms; and R^{a5} indicates a hydrogen atom or an alkyl group having 1 to 5 carbon atoms. As the R^{a4}, a single bond and a straight chain or branched chain alkylene group such as a methylene group, an ethylene group, a propylene group, a tetramethylene group, an ethylethylene group, a pentamethylene group, and a hexamethylene group are preferred. As the R^{a5}, a methyl group and an ethyl group are preferred.

In the alkali-soluble resin, a ratio of a constitutional unit derived from the unsaturated carboxylic acid is preferably 3 to 25 mass%, more preferably 5 to 25 mass%. Also, a ratio of a constitutional unit derived from the epoxy group-containing unsaturated compound is preferably 71 to 95 mass%, more preferably 75 to 90 mass%. Also, a ratio of a constitutional unit derived from the alicyclic group-containing unsaturated compound is preferably 1 to 25 mass%, more preferably 3 to 20 mass%, further preferably 5 to 15 mass%. With the above-specified ranges, it is possible to enhance the adhesiveness of the negative-type photosensitive resin composition to substrates and the strength of the negative-type photosensitive resin composition after curing while maintaining alkali solubility of the obtained resin at an appropriate level.

A mass average molecular weight of the alkali-soluble resin is preferably 1000 to 40000, more preferably 2000 to 30000. With the above-specified range, it is possible to attain satisfactory heat resistance and film strength while attaining favorable developability.

A content of the alkali-soluble resin is preferably 5 to 80 mass%, more preferably 15 to 50 mass%, relative to a solid content of the negative-type photosensitive resin composition. With the above-specified range, there is a tendency that developability is well-balanced.

The photopolymerizable monomer to be contained in the negative-type photosensitive resin composition includes a monofunctional monomer and a multifunctional monomer.

Examples of the monofunctional monomer include (meth)acryl amide, methylol(meth)acrylamide, methoxymethyl(meth)acrylamide, ethoxymethyl(meth)acrylamide, propoxymethyl(meth)acrylamide, butoxymethoxymethyl(meth)acrylamide, N-methylol(meth)acrylamide, N-hydroxymethyl(meth)acrylamide, (meth)acrylic acid, fumaric acid, maleic acid, maleic anhydride, itaconic acid, itaconic anhydride, citraconic acid, citraconic anhydride, crotonic acid, 2-acrylamide-2-methylpropanesulfonic acid, tert-butylacrylamidesulfonic acid, methyl (meth)acrylate, ethyl (meth)acrylate, butyl (meth)acrylate, 2-ethylhexyl (meth)acrylate, cyclohexyl (meth)acrylate, 2-hydroxyethyl (meth)acrylate, 2-hydroxypropyl (meth)acrylate, 2-hydroxybutyl (meth)acrylate, 2-phenoxy-2-hydroxypropyl (meth)acrylate, 2-(meth)acryloyloxy-2-hydroxypropyl phthalate, glycerin mono(meth)acrylate, tetrahydrofurfuryl (meth)acrylate, dimethylamino (meth)acrylate, glycidyl (meth)acrylate, 2,2,2-trifluoroethyl (meth)acrylate, 2,2,3,3-tetrafluoropropyl (meth)acrylate, half (meth)acrylate of a phthalic acid derivative. These monofunctional monomers may be used alone or in combination of two or more kinds thereof.

Meanwhile, examples of the multifunctional monomer include ethylene glycol di(meth)acrylate, diethylene glycol di(meth)acrylate, tetraethylene glycol di(meth)acrylate, propylene glycol di(meth)acrylate, polypropylene glycol di(meth)acrylate, butylene glycol di(meth)acrylate, neopentyl glycol di(meth)acrylate, 1,6-hexane glycol di(meth)acrylate, trimethylolpropane tri(meth)acrylate, glycerin di(meth)acrylate, pentaerythritol triacrylate, pentaerythritol tetraacrylate, dipentaerythritol pentaacrylate, dipentaerythritol hexaacrylate, pentaerythritol di(meth)acrylate, pentaerythritol tri(meth)acrylate, pentaerythritol tetra(meth)acrylate, dipentaerythritol penta(meth)acrylate, dipentaerythritol hexa(meth)acrylate, 2,2-bis(4-(meth)acryloxydiethoxyphenyl)propane, 2,2-bis(4-(meth)acryloxypolyethoxyphenyl)propane, 2-hydroxy-3-(meth)acryloyloxypropyl (meth)acrylate, ethylene glycol diglycidyl ether di(meth)acrylate, diethylene glycol diglycidyl ether di(meth)acrylate, phthalic acid diglycidyl ester di(meth)acrylate, glycerin triacrylate, glycerin polyglycidyl ether poly(meth)acrylate, urethane (meth)acrylate (i.e. tolylene diisocyanate), a reaction product of trimethylhexamethylene diisocyanate, hexamethylene diisocyanate, and 2-hydroxyethyl (meth)acrylate, methylene bis(meth)acrylamide, (meth)acrylamide methylene ether, a multifunctional monomer such as a condensate of a polyvalent alcohol and N-methylol(meth)acrylamide, triacryl formal. These multifunctional monomers may be used alone or in combination of two or more kinds thereof.

A content of the photopolymerizable monomer is preferably 1 to 30 mass%, more preferably 5 to 20 mass%, relative to the solid content of the negative-type photosensitive resin composition. With the above-specified range, there is a tendency that sensitivity, developability, and resolution are well-balanced.

As the photopolymerization initiator to be contained in the negative-type photosensitive resin composition, conventionally known photopolymerization initiators are usable without particular limitation.

Specific examples of the photopolymerization initiator include 1-hydroxy-cyclohexylphenylketone, 2-hydroxy-2-methyl-1-phenylpropan-1-one, 1-[4-(2-hydroxyethoxy)phenyl]-2-hydroxy-2-methyl-1-propan-1-one, 1-(4-isopropylphenyl)-2-hydroxy-2-methylpropan-1-one, 1-(4-dodecylphenyl)-2-hydroxy-2-methylpropan-1-one, 2,2-dimethoxy-1,2-diphenylethan-1-one, bis(4-dimethylaminophenyl)ketone, 2-methyl-1-[4-(methylthio)phenyl]-2-morpholinopropan-1-one, 2-benzyl-2-dimethylamino-1-(4-morpholinophenyl)-butan-1-one, ethanone,1-[9-ethyl-6-(2-methylbenzoyl)-9H-carbozol-3-yl],1-(o-acetyloxime), 2,4,6-trimethylbenzoyldiphenylphosphineoxide, 4-benzoyl-4'-methyldimethylsulfide, 4-dimethylaminobenzoic acid, methyl 4-dimethylaminobenzoate, ethyl 4-dimethylaminobenzoate, butyl 4-dimethylaminobenzoate, 4-dimethylamino-2-ethylhexylbenzoic acid, 4-dimethylamino-2-isoamylbenzoic acid, benzyl-β-methoxyethylacetal, benzyldimethylketal, 1-phenyl-1,2-propanedione-2-(o-ethoxycarbonyl)oxime, methyl o-benzoylbenzoate, 2,4-diethylthioxanthone, 2-chlorothioxanthone, 2,4-dimethylthioxanthone, 1-chloro-4-propoxythioxanthone, thioxanthene, 2-chlorothioxanthene, 2,4-diethylthioxanthene, 2-methylthioxanthene, 2-isopropylthioxanthene, 2-ethylanthraquinone, octamethylanthraquinone, 1,2-benzanthraquinone, 2,3-diphenylanthraquinone, azobisisobutyronitrile, benzoyl peroxide, cumene peroxide, 2-mercaptobenzimidazole, 2-mercaptobenzoxazole, 2-mercaptobenzothiazole, 2-(o-chlorophenyl)-4,5-diphenylimidazole dimers, 2-(o-chlorophenyl)4,5-di(methoxyphenyl)imidazole dimers, 2-(o-fluorophenyl)-4,5-diphenylimidazole dimers, 2-(o-methoxyphenyl)-4,5-diphenylimidazole dimers, 2-(p-methoxyphenyl)-4,5-diphenylimidazole dimers, 2,4,5-triarylimidazole dimers, benzophenone, 2-chlorobenzophenone, 4,4'-bisdiethylaminobenzophenone (i.e. Michler's ketone), 4,4'-bisdiethylaminobenzophenone (i.e. ethyl Michler's ketone), 4,4'-dichlorobenzophenone, 3,3-dimethyl-4-methoxybenzophenone, benzyl, benzoin, benzoin methyl ether, benzoin ethyl ether, benzoin isopropyl ether, benzoin-n-butyl ether, benzoin isobutyl ether, benzoin butyl ether, acetophenone, 2,2-diethoxyacetophenone, p-dimethylacetophenone, p-dimethylaminopropiophenone, dichloroacetophenone, trichloroacetophenone, p-tert-butylacetophenone, p-dimethylaminoacetophenone, p-tert-butyltrichloroacetophenone, p-tert-butyldichloroacetophenone, α,α-dichloro-4-phenoxyacetophenone, thioxanthone, 2-methylthioxanthone, 2-isopropylthioxanthone, dibenzosuberone, pentyl-4-dimethylamino benzoate, 9-phenylacridine, 1,7-bis-(9-acridinyl)heptane, 1,5-bis-(9-acridinyl)pentane, 1,3-bis-(9-acridinyl)propane, p-methoxytriazine, 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-(furan-2-yl)ethenyl-4,6-bis(trichloromethyl)-s-triazine, 2-[2-(4-diethylamino-2-methylphenyl)ethenyl]-4,6-bis(trichloromethyl)-s-triazine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, 2-(4-n-butoxyphenyl)-4,6-bis(trichloromethyl)-s-triazine, 2,4-bistrichloromethyl-6-(3-bromo-4-methoxy)phenyl-s-triazine, 2,4-bis-trichloromethyl-6-(2-bromo-4-methoxy)phenyl-s-triazine, 2,4-bis-trichloromethyl-6-(3-bromo-4-methoxy)styrylphenyl-s-triazine, 2,4-bis-trichloromethyl-6-(2-bromo-4-methoxy) styrylphenyl-s-triazine. Among these, it is particularly preferable to use the oxime-based photopolymerization initiator from the viewpoint of sensitivity. These photopolymerization initiators may be used alone or in combination of two or more kinds thereof.

A content of the photopolymerization initiator is preferably 0.5 to 20 parts by mass relative to 100 parts by mass of the solid content of the negative-type photosensitive resin composition. With the above-specified range, it is possible to attain satisfactory heat resistance and chemical resistance and to suppress a curing defect by improving coating film formation capability.

The negative-type photosensitive composition contains the compound represented by the formula (1) as described above. The compound has favorable solubility to an organic solvent and enables attaining a favorable micropatterning property when contained in the negative-type photosensitive resin composition.

A content of the compound represented by the formula (1) is preferably 0.5 to 95 parts by mass, more preferably 1 to 50 parts by mass, relative to 100 parts by mass of the photopolymerization initiator. With the above-specified range, it is possible to attain a favorable micropatterning property while attaining favorable developability.

The negative-type photosensitive resin composition may further comprise a coloring agent. When the coloring agent is contained, the negative-type photosensitive resin composition is favorably used for forming a color filter of liquid crystal displays, for example. Also, when the negative-type photosensitive resin composition contains a light shielding agent as the coloring agent, it is favorably used for forming a black matrix in the color filter, for example.

The coloring agent is not particularly limited, but it is preferable to use, for example, compounds which are classified into Pigment in Color Index (C.I.; published by The Society of Dyers and Colorist), and specifically those having the following color index (C.I.) numbers.

C.I. pigment yellow 1 (hereinafter, "C.I. pigment yellow" is omitted, and only the numbers are listed), 3, 11, 12, 13, 14, 15, 16, 17, 20, 24, 31, 53, 55, 60, 61, 65, 71, 73, 74, 81, 83, 86, 93, 95, 97, 98, 99, 100, 101, 104, 106, 108, 109, 110, 113, 114, 116, 117, 119, 120, 125, 126, 127, 128, 129, 137, 138, 139, 147, 148, 150, 151, 152, 153, 154, 155, 156, 166, 167, 168, 175, 180, and 185;

C.I. pigment orange 1 (hereinafter, "C.I. pigment orange" is omitted, and only the numbers are listed), 5, 13, 14, 16, 17, 24, 34, 36, 38, 40, 43, 46, 49, 51, 55, 59, 61, 63, 64, 71, and 73;

C.I. pigment violet 1 (hereinafter, "C.I. pigment violet" is omitted, and only the numbers are listed), 19, 23, 29, 30, 32, 36, 37, 38, 39, 40, and 50;

C.I. pigment red 1 (hereinafter, "C.I. pigment red" is omitted, and only the numbers are listed), 2, 3, 4, 5, 6, 7, 8, 9, 10, 11, 12, 14, 15, 16, 17, 18, 19, 21, 22, 23, 30, 31, 32, 37, 38, 40, 41, 42, 48:1, 48:2, 48:3, 48:4, 49:1, 49:2, 50:1, 52:1, 53:1, 57, 57:1, 57:2, 58:2, 58:4, 60:1, 63:1, 63:2, 64:1, 81:1, 83, 88, 90:1, 97, 101, 102, 104, 105, 106, 108, 112, 113, 114, 122, 123, 144, 146, 149, 150, 151, 155, 166, 168, 170, 171, 172, 174, 175, 176, 177, 178, 179, 180, 185, 187, 188, 190, 192, 193, 194, 202, 206, 207, 208, 209, 215, 216, 217, 220, 223, 224, 226, 227, 228, 240, 242, 243, 245, 254, 255, 264, and 265;

C.I. pigment blue 1 (hereinafter, "C.I. pigment blue" is omitted, and only the numbers are listed), 2, 15, 15:3, 15:4, 15:6, 16, 22, 60, 64, and 66;

C.I. pigment green 7, C.I. pigment green 36, and C.I. pigment green 37;

C.I. pigment brown 23, C.I. pigment brown 25, C.I. pigment brown 26, and C.I. pigment brown 28; and

C.I. pigment black 1 and C.I. pigment black 7.

In the case where the light shielding agent is used as the coloring agent, it is preferable to use a black pigment as the light shielding agent. Examples of the black pigment include various types of pigments irrespective of whether it is an organic substance or an inorganic substance, such as carbon black, titanium black, and a metal oxide, a composite oxide, a metal sulfide, a metal sulfate, and a metal carbonate of copper, iron, manganese, cobalt, chromium, nickel, zinc, calcium, silver. Among these, it is preferable to use the carbon black, which has a high light shielding property.

As the carbon black, known carbon black such as channel black, furnace black, thermal black, and lamp black are usable, and it is preferable to use the channel black, which is excellent in light shielding property. Also, a resin-coated carbon black may be used.

Since the resin coated carbon black has lower conductivity than the carbon black without resin coating, it is less subject to electric current leakage when used for black matrixes of liquid crystal display devices and enables producing highly reliable displays with low power consumption.

Each of the above organic pigments may be added as an auxiliary pigment as required in order to adjust a color tone of the carbon black.

Further, a dispersant may be used for uniformly dispersing the coloring agent in the negative-type photosensitive resin composition. As the dispersant, polyethylene imine-based, urethane resin-based, acryl resin-based polymer dispersants is preferably used. Particularly, in the case where the carbon black is used as the coloring agent, it is preferable to use the acryl resin-based dispersant as the dispersant.

Also, the inorganic pigments and the organic pigments may be used alone or in combination, and, in the case of combined use, the organic pigment may be used within the range of 10 to 80 parts by mass, more preferably within the range of 20 to 40 parts by mass, relative to 100 parts by mass in total of the inorganic pigment and the organic pigment.

A content of the coloring agent may appropriately be determined depending on the usage of the negative-type photosensitive resin composition, and, as one example, the content is preferably 5 to 70 parts by mass, more preferably 25 to 60 parts by mass, relative to 100 parts by mass of the solid content of the negative-type photosensitive resin composition.

Particularly, in the case of forming a black matrix by using the negative-type photosensitive resin composition, it is preferable to adjust the amount of the light shielding agent in the negative-type photosensitive resin composition so that an OD value per 1 µm of film thickness of the black matrix is 4 or more. With the OD value of 4 or more per 1 µm of film thickness in the black matrix, it is possible to attain satisfactory display contrast when the negative-type photosensitive resin composition is used for black matrixes of liquid crystal displays.

It is preferable to add to the negative-type photosensitive resin composition the coloring agent as a dispersion which is obtained by dispersing the coloring agent at an appropriate concentration by using the dispersant.

Examples of the organic solvent in the negative-type photosensitive resin composition include (poly)alkylene glycol monoalkyl ethers such as ethylene glycol monomethyl ether, ethylene glycol monoethyl ether, ethylene glycol-n-propyl ether, ethylene glycol mono-n-butyl ether, diethylene glycol monomethyl ether, diethylene glycol monoethyl ether, diethylene glycol mono-n-propyl ether, diethylene glycol mono-n-butyl ether, triethylene glycol monomethyl ether, triethylene glycol monoethyl ether, propylene glycol monomethyl ether, propylene glycol monoethyl ether, propylene glycol mono-n-propyl ether, propylene glycol mono-n-butyl ether, dipropylene glycol monomethyl ether, dipropylene glycol monoethyl ether, dipropylene glycol mono-n-propyl ether, dipropylene glycol mono-n-butyl ether, tripropylene glycol monomethyl ether, and tripropylene glycol monoethyl ether; (poly)alkylene glycol monoalkyl ether acetates such as ethylene glycol monomethyl ether acetate, ethylene glycol monoethyl ether acetate, diethylene glycol monomethyl ether acetate, diethylene glycol monoethyl ether acetate, propylene glycol monomethyl ether acetate, and propylene glycol monoethyl ether acetate; other ethers such as diethylene glycol dimethyl ether, diethylene glycol methylethyl ether, diethylene glycol diethyl ether, and tetrahydrofuran; ketones such as methyl ethyl ketone, cyclohexanone, 2-heptanone, and 3-heptanone; alkyl lactates such as methyl 2-hydroxypropionate and ethyl 2-hydroxypropionate; other esters such as ethyl 2-hydroxy-2-methylpropionate, methyl 3-methoxypropionate, ethyl 3-methoxy propionate, methyl 3-ethoxypropionate, ethyl 3-ethoxypropionate, ethyl ethoxyacetate, ethyl hydroxyacetate, methyl 2-hydroxy-3-methylbutanoate, 3-methyl-3-methoxybutylacetate, 3-methyl-3-methoxybutylpropionate, ethyl acetate, n-propyl acetate, isopropyl acetate, n-butyl acetate, isobutyl acetate, n-pentyl formate, isopentyl acetate, n-butyl propionate, ethyl butyrate, n-propyl butyrate, isopropyl butyrate, n-butyl butyrate, methyl pyruvate, ethyl pyruvate, n-propyl pyruvate, methyl acetoacetate, ethyl acetoacetate, and ethyl 2-oxobutanoate; aromatic hydrocarbons such as toluene and xylene; amides such as N-methylpyrrolidone, N,N-dimethylformamide, and N,N-dimethylacetamide. The organic solvents may be used alone or in combination of two or more kinds thereof.

Among the above organic solvents, propylene glycol monomethyl ether, ethylene glycol monomethyl ether acetate, propylene glycol monomethyl ether acetate, propyleneglycol monoethyl ether acetate, diethylene glycol dimethyl ether, diethylene glycol methylethyl ether, cyclohexanone, 3-methoxybutyl acetate are preferred since they exhibit excellent solubility with respect to the alkali-soluble resin, the photopolymerizable monomer, the photopolymerization initiator, and the compound represented by the formula (1) and improve a dispersing property of the coloring agent, and it is particularly preferable to use propylene glycol monomethyl ether acetate or 3-methoxybutyl acetate.

A content of the organic solvent is preferably such that a solid content concentration of the negative-type photosensitive resin composition is 1 to 50 mass%, more preferably 5 to 30 mass%.

The negative-type photosensitive resin composition may contain various additives as required. Examples of the additives include a sensitizer, a curing accelerator, a filler, an adhesion accelerator, an antioxidant, an ultraviolet ray absorber, a flocculation inhibitor, thermal polymerization inhibitor, an anti-foaming agent, a surfactant, and the like.

The negative-type photosensitive resin composition is prepared by mixing each of the above-described components with a stirring machine. In order that the prepared negative-type photosensitive resin composition becomes homogenous, the negative-type photosensitive resin composition may be filtered using a membrane filter.

### EXAMPLES

Hereinafter, the present invention will be described more specifically with examples, but the scope of the present invention is not limited to these examples.
Compounds represented by the Formula (1) and Comparative Compounds

Compounds 1 to 20 represented by the following formulas were prepared as the compounds represented by the formula (1). Synthesis methods of the compounds 1 to 20 are described below. Also, for the purpose of comparison, Comparative Compounds 1 to 11 represented by the following formulas were prepared.

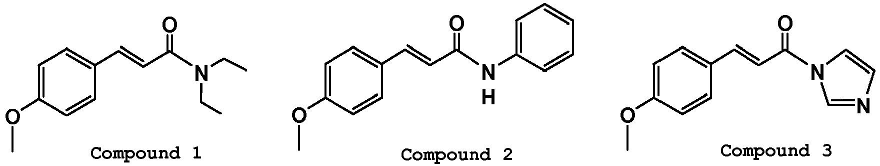

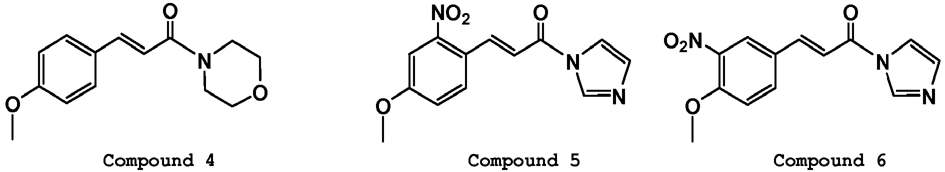

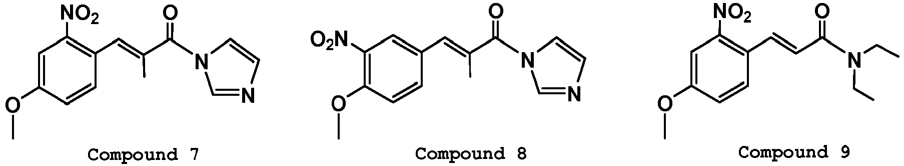

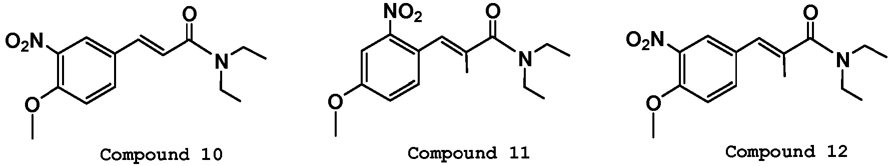

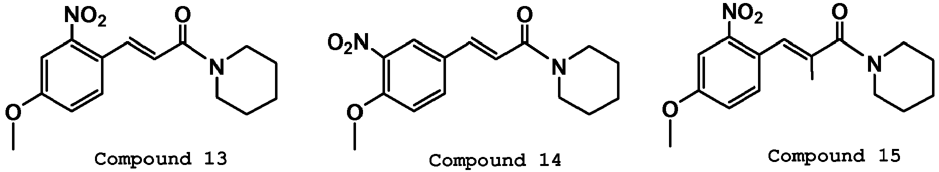

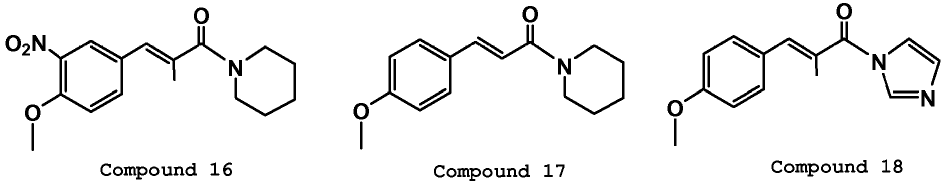

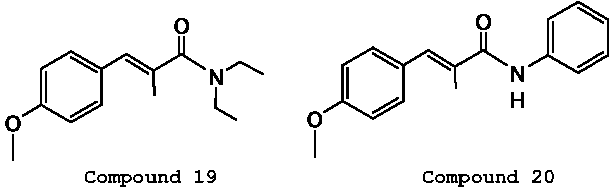

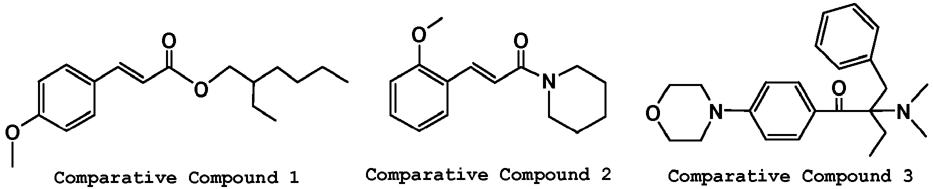

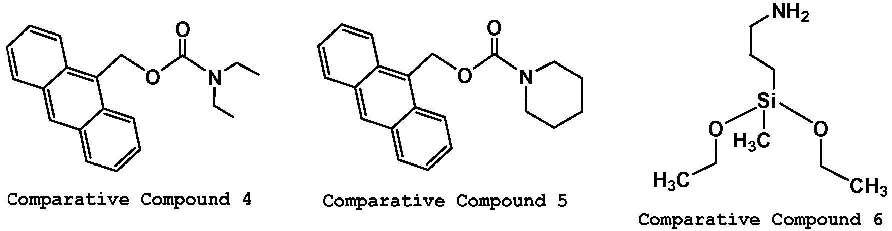

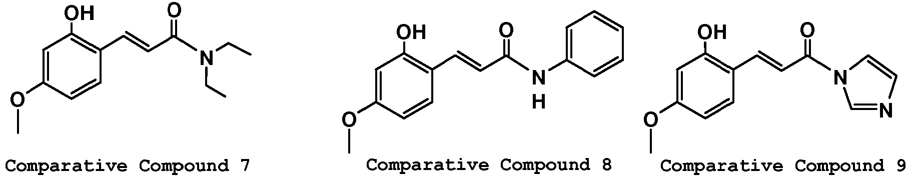

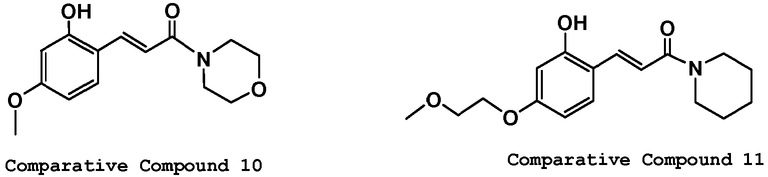

### [Synthesis Method of Compound 1]

5.90 g (30 mmol) of 3-(4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 1 (4.65 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 2]

5.90 g (30 mmol) of 3-(4-methoxyphenyl)acrylic acid chloride was dissolved in 50 ml of dry ether, and then 4.59 ml (equivalence ratio: 1.1) of triethylamine and 3.07 ml (equivalence ratio: 1.1) of aniline were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 2 (6.31 g, 25 mmol) was obtained by performing purification by column chromatography using hexane-ethyl acetate as a developing solvent and silica gel as a supporting carrier. Yield based on acrylic acid chloride was 83%.

### [Synthesis Method of Compound 3]

5.90 g (30 mmol) of 3-(4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 3 (3.41 g, 15 mmol) was obtained by performing purification by column chromatography using hexane-ethyl acetate as a developing solvent and silica gel as a supporting carrier. Yield based on acrylic acid chloride was 50%.

### [Synthesis Method 1 of Compound 4]

5.90 g (30 mmol) of 3-(4-methoxyphenyl)acrylic acid chloride was dissolved in 50 ml of dry ether, and then 4.59 ml (equivalence ratio: 1.1) of triethylamine and 2.25 ml (equivalence ratio: 1.1) of morpholine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 4 (3.41 g, 15 mmol) was obtained by performing purification by column chromatography using hexane-ethyl acetate as a developing solvent and silica gel as a supporting carrier. Yield based on acrylic acid chloride was 50%.

### [Synthesis Method 2 of Compound 4]

10.78 g (30 mmol) of 4-methoxycinnamic acid p-nitrophenyl ester was dissolved in 50 ml of dry ether, and then 2.25 ml (equivalence ratio: 1.1) of morpholine was added, followed by stirring for 4 hours at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 4 (6.62 g, 15 mmol) was obtained by performing purification by column chromatography using hexane-ethyl acetate as a developing solvent and silica gel as a supporting carrier. Yield based on cinnamic acid phenyl ester was 97%.

### [Synthesis Method of Compound 5]

7.25 g (30 mmol) of 3-(2-nitro-4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 5 (4.08 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 6]

7.25 g (30 mmol) of 3-(3-nitro-4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 6 (4.08 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 7]

7.67 g (30 mmol) of 2-methyl-3-(2-nitro-4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 7 (4.29 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 8]

7.67 g (30 mmol) of 2-methyl-3-(3-nitro-4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 8 (3.41 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 9]

7.25 g (30 mmol) of 3-(2-nitro-4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 9 (5.55 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 10]

7.25 g (30 mmol) of 3-(3-nitro-4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 10 (5.55 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 11]

7.67 g (30 mmol) of 2-methyl-3-(2-nitro-4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 11 (5.83 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 12]

7.67 g (30 mmol) of 2-methyl-3-(3-nitro-4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 12 (5.83 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 13]

7.25 g (30 mmol) of 3-(2-nitro-4-methoxyphenyl)acrylic acid chloride was dissolved in 50 ml of dry ether, and then 4.59 ml (equivalence ratio: 1.1) of triethylamine and 2.81 ml (equivalence ratio: 1.1) of piperidine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 13 (5.62 g, 23 mmol) was obtained by performing purification by column chromatography using hexane-ethyl acetate as a developing solvent and silica gel as a supporting carrier. Yield based on acrylic acid chloride was 77%.

### [Synthesis Method of Compound 14]

7.25 g (30 mmol) of 3-(3-nitro-4-methoxyphenyl)acrylic acid chloride was dissolved in 50 ml of dry ether, and then 4.59 ml (equivalence ratio: 1.1) of triethylamine and 2.81 ml (equivalence ratio: 1.1) of piperidine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 14 (5.62 g, 23 mmol) was obtained by performing purification by column chromatography using hexane-ethyl acetate as a developing solvent and silica gel as a supporting carrier. Yield based on acrylic acid chloride was 67%.

### [Synthesis Method of Compound 15]

7.67 g (30 mmol) of 2-methyl-3-(2-nitro-4-methoxyphenyl)acrylic acid chloride was dissolved in 50 ml of dry ether, and then 4.59 ml (equivalence ratio: 1.1) of triethylamine and 2.81 ml (equivalence ratio: 1.1) of piperidine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 15 (5.83 g, 23 mmol) was obtained by performing purification by column chromatography using hexane-ethyl acetate as a developing solvent and silica gel as a supporting carrier. Yield based on acrylic acid chloride was 67%.

### [Synthesis Method of Compound 16]

7.67 g (30 mmol) of 2-methyl-3-(3-nitro-4-methoxyphenyl)acrylic acid chloride was dissolved in 50 ml of dry ether, and then 4.59 ml (equivalence ratio: 1.1) of triethylamine and 2.81 ml (equivalence ratio: 1.1) of piperidine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 16 (5.83 g, 23 mmol) was obtained by performing purification by column chromatography using hexane-ethyl acetate as a developing solvent and silica gel as a supporting carrier. Yield based on acrylic acid chloride was 67%.

### [Synthesis Method of Compound 17]

5.90 g (30 mmol) of 3-(4-methoxyphenyl)acrylic acid chloride was dissolved in 50 ml of dry ether, and then 4.59 ml (equivalence ratio: 1.1) of triethylamine and 2.81 ml (equivalence ratio: 1.1) of piperidine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 17 (3.41 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 18]

6.32 g (30 mmol) of 2-methyl-3-(4-methoxyphenyl)acrylic acid chloride was dissolved in 50 ml of dry ether, and then 4.59 ml (equivalence ratio: 1.1) of triethylamine and 2.25 ml (equivalence ratio: 1.1) of imidazole were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 18 (3.62 g, 15 mmol) was obtained by performing purification by column chromatography using hexane-ethyl acetate as a developing solvent and silica gel as a supporting carrier. Yield based on acrylic acid chloride was 50%.

### [Synthesis Method of Compound 19]

6.32 g (30 mmol) of 2-methyl-3-(4-methoxyphenyl)acrylic acid chloride was dissolved in 50 ml of dry ether, and then 4.59 ml (equivalence ratio: 1.1) of triethylamine and 2.41 ml (equivalence ratio: 1.1) of diethylamine were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 19 (4.93 g, 20 mmol) was obtained by performing purification by column chromatography using hexane-ethyl acetate as a developing solvent and silica gel as a supporting carrier. Yield based on acrylic acid chloride was 67%.

### [Synthesis Method of Compound 20]

6.32 g (30 mmol) of 2-methyl-3-(4-methoxyphenyl)acrylic acid chloride was dissolved in 50 ml of dry ether, and then 4.59 ml (equivalence ratio: 1.1) of triethylamine and 3.07 ml (equivalence ratio: 1.1) of aniline were added, followed by stirring for 1 hour at room temperature. After washing with 50 ml of water, 50 ml of a saturated NaHCO₃ aqueous solution, and 1N hydrochloric acid, drying with magnesium sulfate was performed, followed by concentration under a reduced pressure. Corresponding Compound 20 (4.29 g, 25 mmol) was obtained by performing purification by column chromatography using hexane-ethyl acetate as a developing solvent and silica gel as a supporting carrier. Yield based on acrylic acid chloride was 83%.

### [Evaluation]

For each of Compounds 1 to 20 and Comparative Compounds 1 to 10, a wavelength (λₘₐₓ) at an absorption spectrum peak and a gram absorption coefficient at λₘₐₓ were measured. Also, solubility of Compounds 1 to 20 and Comparative Compounds 1 to 10 to propylene glycol monomethyl ether acetate (PM) and cyclohexanone (AN) were confirmed. The results are shown in Table 1 below.

**[Table 1]**

| | λₘₐₓ (nm) | Gram Absorption Coefficient at λₘₐₓ | Solubility to Solvent | |
|---|---|---|---|---|
| | | | PM | AN |
| Compound 1 | 309 | 90 | >50 mass% | >50 mass% |
| Compound 2 | 317 | 117 | >50 mass% | >50 mass% |
| Compound 3 | 293 | 81 | >50 mass% | >50 mass% |
| Compound 4 | 310 | 79 | >50 mass% | >50 mass% |
| Compound 5 | 332 | 121 | >50 mass% | >50 mass% |
| Compound 6 | 344 | 86 | >50 mass% | >50 mass% |
| Compound 7 | 339 | 137 | >50 mass% | >50 mass% |
| Compound 8 | 351 | 98 | >50 mass% | >50 mass% |
| Compound 9 | 342 | 102 | >50 mass% | >50 mass% |
| Compound 10 | 354 | 119 | >50 mass% | >50 mass% |
| Compound 11 | 349 | 107 | >50 mass% | >50 mass% |
| Compound 12 | 361 | 127 | >50 mass% | >50 mass% |
| Compound 13 | 313 | 87 | >50 mass% | >50 mass% |
| Compound 14 | 322 | 94 | >50 mass% | >50 mass% |
| Compound 15 | 313 | 92 | >50 mass% | >50 mass% |
| Compound 16 | 323 | 105 | >50 mass% | >50 mass% |
| Compound 17 | 281 | 74 | >50 mass% | >50 mass% |
| Compound 18 | 301 | 84 | >50 mass% | >50 mass% |
| Compound 19 | 318 | 86 | >50 mass% | >50 mass% |
| Compound 20 | 323 | 79 | >50 mass% | >50 mass% |
| Comparative Compound 1 | 309 | 78 | >50 mass% | >50 mass% |
| Comparative Compound 2 | 275 | 76 | <1 mass% | <1 mass% |
| Comparative Compound 3 | 321 | 53 | >30 mass% | >50 mass% |
| Comparative Compound 4 | 255 | 477 | >50 mass% | >50 mass% |
| Comparative Compound 5 | 255 | 464 | >50 mass% | >50 mass% |
| Comparative Compound 6 | 212 | 143 | >50 mass% | >50 mass% |
| Comparative Compound 7 | 304 | 72 | >50 mass% | >50 mass% |
| Comparative Compound 8 | 315 | 96 | >50 mass% | >50 mass% |
| Comparative Compound 9 | 290 | 65 | >50 mass% | >50 mass% |
| Comparative Compound 10 | 305 | 58 | >50 mass% | >50 mass% |

As is apparent from Table 1, each of Compounds 1 to 20 had a favorable solubility to propylene glycol monomethyl ether acetate (PM) and cyclohexanone (AN).

### Preparation of Negative-Type Photosensitive Resin Composition

### Example 1

All of the following components were mixed and then dissolved in a mixed solvent with a mass ratio of 3-methoxybutyl acetate (MA)/propylene glycol monomethyl ether acetate (PM)/cyclohexanone (AN) = 60/20/20 to prepare a negative-type photosensitive resin composition having a solid content concentration of 15 mass%.
- Alkali-Soluble Resin
   Resin (A-1) (solid content: 55%; solvent: 3-methoxybutyl acetate) : 310 parts by mass
- Photopolymerizable Monomer
   Dipentaerythritol hexaacrylate (DPHA) : 65 parts by mass
   • Photopolymerization Initiator
   "OXE-02" (trade name; manufactured by BASF Corporation) : 15 parts by mass
- Compound represented by the Formula (1)
   Compound 1 described above : 5 parts by mass
- Coloring Agent

A carbon dispersion "CF Black" (trade name; manufactured by Mikuni Color Ltd.; solid content: 25%; solvent: 3-methoxybutyl acetate) : 1200 parts by mass

Synthesis method of the resin (A-1) is as follows.

A 500-ml 4-necked flask was charged with 235 g of a bisphenol fluorine-type epoxy resin (epoxy equivalent: 235), 110 mg of tetramethylammonium chloride, 100 mg of 2,6-di-tert-butyl-4-methylphenol, and 72.0 g of acrylic acid, and the components were heated and dissolved at 90°C to 100°C while blowing the air into the flask at a rate of 25 ml/min. Next, the solution in the state of white turbidity was gradually heated to 120°C so as to achieve complete dissolution. In this step, the solution gradually became transparent and viscous, but stirring was performed continually as before. An acid value was measured during the stirring, and the heating with stirring was continued until the acid value fell below 1.0 mgKOH/g. It took 12 hours to reach the target acid value. After that, cooling to room temperature was performed to obtain bisphenol fluorine-type epoxy acrylate represented by the following formula (a-4) which was in the form of a colorless and transparent solid.

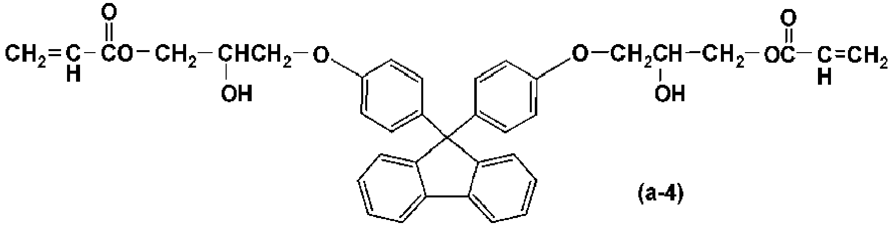

Subsequently, after dissolving 307.0 g of the thus-obtained bisphenol fluorine-type epoxy acrylate by adding thereto 600 g of 3-methoxybutyl acetate, 80.5 g of benzophenonetetracarboxylic acid dianhydride and 1 g of tetraethyl ammonium bromide were mixed, followed by gradually heating to allow a reaction to take place at 110°C to 115°C for 4 hours. After confirming disappearance of the acid anhydride group, 38.0 g of 1,2,3,6-tetrahydrophthalic acid anhydride was mixed, and a reaction was allowed to take place at 90°C for 6 hours to obtain the resin (A-1). The disappearance of the acid anhydride group was confirmed by IR spectrum.

The resin (A-1) is equivalent to the compound represented by the formula (a-1).

### Examples 2 to 20 and Comparative Examples 1 to 10

Negative-type photosensitive resin compositions were prepared in the same manner as Example 1, except that Compounds 2 to 20 and Comparative Compounds 1 to 10 described above, respectively, were used instead of Compound 1.

### Evaluation

The negative-type photosensitive resin compositions of Examples 1 to 20 and Comparative Examples 1 to 10 were coated onto a glass substrate (100 mm × 100 mm) by using a spin coater, and pre-baking was performed at 90°C for 120 seconds to form coating films having a film thickness of 1.0 µm. Next, the coating film was irradiated with ultraviolet rays via a negative mask in which a 20-µm line pattern was formed, by using a mirror projection aligner (product name: TME-150RTO, manufactured by Topcon Corporation) with an exposure gap of 50 µm. The light exposures were set to four levels of 20, 40, 60, and 120 J/cm². The coating film after the exposure was developed in a 0.04-mass% KOH aqueous solution at 26°C for 40 seconds and then was post-baked at 230°C for 30 minutes, thereby forming a line pattern.

Likewise, the coating film was irradiated with ultraviolet rays via negative masks in each of which a 2-, 5-, 10-, or 20-µm line pattern was formed, at an exposure gap of 50 µm. The light exposure was set to 10 mJ/cm². The coating film after the exposure was developed in a 0.04-mass% KOH aqueous solution at 26°C for 40 seconds and then was post-baked at 230°C for 30 minutes, thereby forming a line pattern.

An OD value per 1 µm of the film thickness of each of the formed line patterns was measured by using an OD measurement device, D-200II (product of GretagMacbeth LLC).

Also, the line pattern was observed with an optical microscope to evaluate the pattern straightness. The pattern straightness was evaluated as "good" if there were no curves or irregularities on the line edges, and as "poor" if there were curves or irregularities.

Further, pattern adhesion was evaluated by observing the line pattern with an optical microscope. The pattern adhesion was evaluated as "good" if the line pattern was formed without detachment from the substrate, and as "none" if the line pattern was not formed due to detachment from the substrate.

Further, the existence of residues in the unexposed portions after the development was evaluated.

The results are shown in Tables 2 to 5 below.

**[Table 2]**

| | Compound of Formula (1) | OD Value | Pattern Straightness | | | |
|---|---|---|---|---|---|---|
| | | | 20mJ | 40mJ | 60mJ | 120mJ |
| Example 1 | Compound 1 | 4.5 | Good | Good | Good | Good |
| Example 2 | Compound 2 | 4.5 | Good | Good | Good | Good |
| Example 3 | Compound 3 | 4.5 | Good | Good | Good | Good |
| Example 4 | Compound 4 | 4.5 | Good | Good | Good | Good |
| Example 5 | Compound 5 | 4.5 | Good | Good | Good | Good |
| Example 6 | Compound 6 | 4.5 | Good | Good | Good | Good |
| Example 7 | Compound 7 | 4.5 | Good | Good | Good | Good |
| Example 8 | Compound 8 | 4.5 | Good | Good | Good | Good |
| Example 9 | Compound 9 | 4.5 | Good | Good | Good | Good |
| Example 10 | Compound 10 | 4.5 | Good | Good | Good | Good |
| Example 11 | Compound 11 | 4.5 | Good | Good | Good | Good |
| Example 12 | Compound 12 | 4.5 | Good | Good | Good | Good |
| Example 13 | Compound 13 | 4.5 | Good | Good | Good | Good |
| Example 14 | Compound 14 | 4.5 | Good | Good | Good | Good |
| Example 15 | Compound 15 | 4.5 | Good | Good | Good | Good |
| Example 16 | Compound 16 | 4.5 | Good | Good | Good | Good |
| Example 17 | Compound 17 | 4.5 | Good | Good | Good | Good |
| Example 18 | Compound 18 | 4.5 | Good | Good | Good | Good |
| Example 19 | Compound 19 | 4.5 | Good | Good | Good | Good |
| Example 20 | Compound 20 | 4.5 | Good | Good | Good | Good |

**[Table 3]**

| | Compound of Formula (1) | Pattern Adhesion | | | | Residue |
|---|---|---|---|---|---|---|
| | | 2µm | 5µm | 10µm | 20µm | |
| Example 1 | Compound 1 | Good | Good | Good | Good | None |
| Example 2 | Compound 2 | Good | Good | Good | Good | None |
| Example 3 | Compound 3 | Good | Good | Good | Good | None |
| Example 4 | Compound 4 | Good | Good | Good | Good | None |
| Example 5 | Compound 5 | Good | Good | Good | Good | None |
| Example 6 | Compound 6 | Good | Good | Good | Good | None |
| Example 7 | Compound 7 | Good | Good | Good | Good | None |
| Example 8 | Compound 8 | Good | Good | Good | Good | None |
| Example 9 | Compound 9 | Good | Good | Good | Good | None |
| Example 10 | Compound 10 | Good | Good | Good | Good | None |
| Example 11 | Compound 11 | Good | Good | Good | Good | None |
| Example 12 | Compound 12 | Good | Good | Good | Good | None |
| Example 13 | Compound 13 | Good | Good | Good | Good | None |
| Example 14 | Compound 14 | Good | Good | Good | Good | None |
| Example 15 | Compound 15 | Good | Good | Good | Good | None |
| Example 16 | Compound 16 | Good | Good | Good | Good | None |
| Example 17 | Compound 17 | Good | Good | Good | Good | None |
| Example 18 | Compound 18 | Good | Good | Good | Good | None |
| Example 19 | Compound 19 | Good | Good | Good | Good | None |
| Example 20 | Compound 20 | Good | Good | Good | Good | None |

**[Table 4]**

| | Comparative Compound | OD Value | Pattern Straightness | | | |
|---|---|---|---|---|---|---|
| | | | 20mJ | 40mJ | 60mJ | 120mJ |
| Comparative Example 1 | Comparative Compound 1 | 4.5 | Poor | Poor | Good | Good |
| Comparative Example 2 | Comparative Compound 2 | 4.5 | Poor | Poor | Good | Good |
| Comparative Example 3 | Comparative Compound 3 | 4.5 | Poor | Good | Good | Good |
| Comparative Example 4 | Comparative Compound 4 | 4.5 | Poor | Poor | Poor | Good |
| Comparative Example 5 | Comparative Compound 5 | 4.5 | Poor | Poor | Poor | Good |
| Comparative Example 6 | Comparative Compound 6 | 4.5 | Poor | Poor | Poor | Good |
| Comparative Example 7 | Comparative Compound 7 | 4.5 | Poor | Good | Good | Good |
| Comparative Example 8 | Comparative Compound 8 | 4.5 | Poor | Good | Good | Good |
| Comparative Example 9 | Comparative Compound 9 | 4.5 | Poor | Good | Good | Good |
| Comparative Example 10 | Comparative Compound 10 | 4.5 | Poor | Good | Good | Good |

**[Table 5]**

| | Comparative Compound | Pattern Adhesion | | | | Residue |
|---|---|---|---|---|---|---|
| | | 2µm | 5µm | 10µm | 20µm | |
| Comparative Example 1 | Comparative Compound 1 | None | None | None | Good | None |
| Comparative Example 2 | Comparative Compound 2 | None | None | None | Good | None |
| Comparative Example 3 | Comparative Compound 3 | None | None | Good | Good | Existing |
| Comparative Example 4 | Comparative Compound 4 | None | None | None | None | None |
| Comparative Example 5 | Comparative Compound 5 | None | None | None | None | None |
| Comparative Example 6 | Comparative Compound 6 | None | None | None | None | Existing |
| Comparative Example 7 | Comparative Compound 7 | None | None | Good | Good | None |
| Comparative Example 8 | Comparative Compound 8 | None | None | Good | Good | None |
| Comparative Example 9 | Comparative Compound 9 | None | None | Good | Good | None |
| Comparative Example 10 | Comparative Compound 10 | None | None | Good | Good | None |

As can be understood from Tables 2 and 3, in the case where the negative-type photosensitive resin compositions of Examples 1 to 20 containing Compounds 1 to 20 represented by the formula (1) were used, it was possible to form a line pattern excellent in straightness even at a low light exposure of 20 mJ/cm². Also, a 2-µm line pattern was in close contact with the substrate even at a low light exposure of 10 mJ/cm². Further, in the case where the negative-type photosensitive resin compositions of Examples 1 to 20 were used, no development residue was present.

In contrast, in the case where the negative-type photosensitive resin compositions of Comparative Examples 1 to 10 containing Comparative Compounds 1 to 10 were used, as can be understood from Tables 4 and 5, both of the pattern straightness and the pattern adhesion were inferior to Examples 1 to 20, and a favorable micropatterning property was not attained.

Particularly, Comparative Compound 6 contained in the negative-type photosensitive resin composition of Comparative Example 6 is an amine-based silane coupling agent, which is known as an adhesion enhancer, but even a 20-µm line pattern failed to be in close contact with the substrate at a low light exposure of 10 mJ/cm².

Also, Comparative Compounds 7 to 10 contained in the photosensitive resin compositions of Comparative Examples 7 to 10 are ones in which a hydroxyl group is bonded to the ortho position of the benzene ring of Compounds 1 to 4, and only a line pattern having an inferior straightness was formed at a low light exposure of 20 mJ/cm². Also, only line patterns having the width of 10 µm or more were in close contact with the substrate at a low light exposure of 10 mJ/cm².

## Claims

1. A use of a compound represented by the following formula (1): 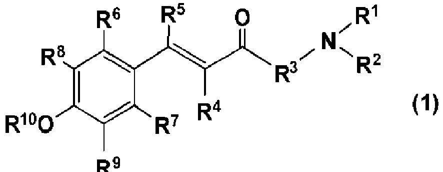
wherein R¹ and R² each independently indicate a hydrogen atom or an organic group,
provided that at least one of R¹ and R² indicates an organic group;
R¹ and R² may be bonded to form a ring structure and may contain a hetero atom bond;
R³ indicates a single bond or an organic group;
R⁴ and R⁵ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group;
R⁶ and R⁷ each independently indicate a hydrogen atom, a halogen atom, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group,
R⁸ and R⁹ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group, or an organic group;
two or more of R⁶, R⁷, R⁸, and R⁹ may be bonded to form a ring structure and may contain a hetero atom bond; and
R¹⁰ indicates an alkyl group having 1 to 12 carbons ,
as a base generator.

2. A use of a compound represented by the following formula (1): 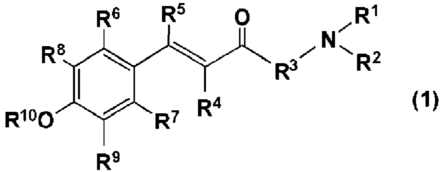
wherein R¹ and R² each independently indicate a hydrogen atom or an organic group,
provided that at least one of R¹ and R² indicates an organic group;
R¹ and R² may be bonded to form a ring structure and may contain a hetero atom bond;
R³ indicates a single bond or an organic group;
R⁴ and R⁵ each independently indicate a hydrogen atom, a halogen atom, a hydroxyl group, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, or an organic group;
R⁶, R⁷, R⁸, and R⁹ each independently indicate a hydrogen atom, a halogen atom, a mercapto group, a sulfide group, a silyl group, a silanol group, a nitro group, a nitroso group, a sulfino group, a sulfo group, a sulfonato group, a phosphino group, a phosphinyl group, a phosphono group, a phosphonato group, an amino group, an ammonio group, or an organic group,
two or more of R⁶, R⁷, R⁸, and R⁹ may be bonded to form a ring structure and may contain a hetero atom bond; and
R¹⁰ indicates a hydrogen atom, or an alkyl group having 1 to 12 carbons,
as an adhesion enhancer.

## Patentansprüche

1. Verwendung einer Verbindung, dargestellt durch die folgende Formel (1): 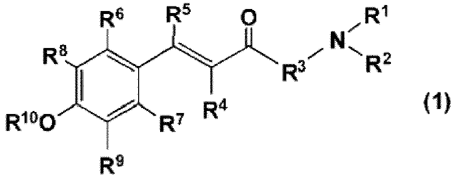
worin R¹ und R² jeweils unabhängig voneinander ein Wasserstoffatom oder eine organische Gruppe bezeichnen,
unter der Voraussetzung, dass mindestens eines von R¹ und R² eine organische Gruppe bezeichnet;
R¹ und R² unter Bildung einer Ringstruktur gebunden sein können und eine Heteroatom-Bindung enthalten können;
R³ eine Einfachbindung oder eine organische Gruppe bezeichnet;
R⁴ und R⁵ jeweils unabhängig voneinander ein Wasserstoffatom, ein Halogenatom, eine Hydroxylgruppe, eine Mercaptogruppe, eine Sulfidgruppe, eine Silylgruppe, eine Silanolgruppe, eine Nitrogruppe, eine Nitrosogruppe, eine Sulfinogruppe, eine Sulfogruppe, eine Sulfonatogruppe, eine Phosphinogruppe, eine Phosphinylgruppe, eine Phosphonogruppe, eine Phosphonatogruppe oder eine organische Gruppe bezeichnen;
R⁶ und R⁷ jeweils unabhängig voneinander ein Wasserstoffatom, ein Halogenatom, eine Mercaptogruppe, eine Sulfidgruppe, eine Silylgruppe, eine Silanolgruppe, eine Nitrogruppe, eine Nitrosogruppe, eine Sulfinogruppe, eine Sulfogruppe, eine Sulfonatogruppe, eine Phosphinogruppe, eine Phosphinylgruppe, eine Phosphonogruppe, eine Phosphonatogruppe, eine Aminogruppe, eine Ammoniogruppe bezeichnen,
R⁸ und R⁹ jeweils unabhängig voneinander ein Wasserstoffatom, ein Halogenatom, eine Hydroxylgruppe, eine Mercaptogruppe, eine Sulfidgruppe, eine Silylgruppe, eine Silanolgruppe, eine Nitrogruppe, eine Nitrosogruppe, eine Sulfinogruppe, eine Sulfogruppe, eine Sulfonatogruppe, eine Phosphinogruppe, eine Phosphinylgruppe, eine Phosphonogruppe, eine Phosphonatogruppe, eine Aminogruppe, eine Ammoniogruppe oder eine organische Gruppe bezeichnen,
zwei oder mehr von R⁶, R⁷, R⁸ und R⁹ unter Bildung einer Ringstruktur gebunden sein können und eine Heteroatom-Bindung enthalten können; und
R¹⁰ eine Alkylgruppe mit 1 bis 12 Kohlenstoffen bezeichnet,
als Basenbildner.

2. Verwendung einer Verbindung, dargestellt durch die folgende Formel (1): 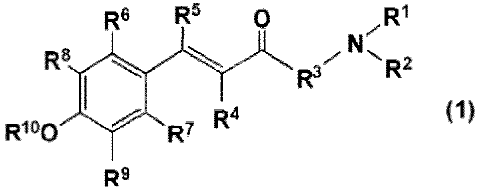
worin R¹ und R² jeweils unabhängig voneinander ein Wasserstoffatom oder eine organische Gruppe bezeichnen,
unter der Voraussetzung, dass mindestens eines von R¹ und R² eine organische Gruppe bezeichnet;
R¹ und R² unter Bildung einer Ringstruktur gebunden sein können und eine Heteroatom-Bindung enthalten können;
R³ eine Einfachbindung oder eine organische Gruppe bezeichnet;
R⁴ und R⁵ jeweils unabhängig voneinander ein Wasserstoffatom, ein Halogenatom, eine Hydroxylgruppe, eine Mercaptogruppe, eine Sulfidgruppe, eine Silylgruppe, eine Silanolgruppe, eine Nitrogruppe, eine Nitrosogruppe, eine Sulfinogruppe, eine Sulfogruppe, eine Sulfonatogruppe, eine Phosphinogruppe, eine Phosphinylgruppe, eine Phosphonogruppe, eine Phosphonatogruppe oder eine organische Gruppe bezeichnen;
R⁶, R⁷, R⁸ und R⁹ jeweils unabhängig voneinander ein Wasserstoffatom, ein Halogenatom, eine Mercaptogruppe, eine Sulfidgruppe, eine Silylgruppe, eine Silanolgruppe, eine Nitrogruppe, eine Nitrosogruppe, eine Sulfinogruppe, eine Sulfogruppe, eine Sulfonatogruppe, eine Phosphinogruppe, eine Phosphinylgruppe, eine Phosphonogruppe, eine Phosphonatogruppe, eine Aminogruppe, eine Ammoniogruppe oder eine organische Gruppe bezeichnen,
zwei oder mehr von R⁶, R⁷, R⁸ und R⁹ unter Bildung einer Ringstruktur gebunden sein können und eine Heteroatom-Bindung enthalten können; und
R¹⁰ ein Wasserstoffatom oder eine Alkylgruppe mit 1 bis 12 Kohlenstoffen bezeichnet,
als Haftungsverstärker.

## Revendications

1. Utilisation d'un composé représenté par la formule (1) suivante : 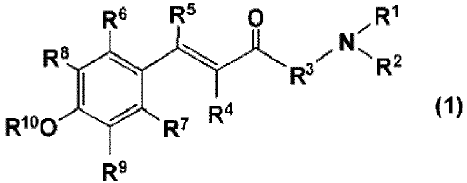
où R¹ et R² indiquent chacun indépendamment un atome d'hydrogène ou un groupe organique, à condition qu'au moins l'un de R¹ et R² indique un groupe organique ;
R¹ et R² peuvent être liés pour former une structure cyclique et peuvent contenir une liaison hétéroatome ;
R³ indique une simple liaison ou un groupe organique ;
R⁴ et R⁵ indiquent chacun indépendamment un atome d'hydrogène, un atome d'halogène, un groupe hydroxyle, un groupe mercapto, un groupe sulfure, un groupe silyle, un groupe silanol, un groupe nitro, un groupe nitroso, un groupe sulfino, un groupe sulfo, un groupe sulfonato, un groupe phosphino, un groupe phosphinyle, un groupe phosphono, un groupe phosphonato, ou un groupe organique ;
R⁶ et R⁷ indiquent chacun indépendamment un atome d'hydrogène, un atome d'halogène, un groupe mercapto, un groupe sulfure, un groupe silyle, un groupe silanol, un groupe nitro, un groupe nitroso, un groupe sulfino, un groupe sulfo, un groupe sulfonato, un groupe phosphino, un groupe phosphinyle, un groupe phosphono, un groupe phosphonato, un groupe amino, un groupe ammonio,
R⁸ et R⁹ indiquent chacun indépendamment un atome d'hydrogène, un atome d'halogène, un groupe hydroxyle, un groupe mercapto, un groupe sulfure, un groupe silyle, un groupe silanol, un groupe nitro, un groupe nitroso, un groupe sulfino, un groupe sulfo, un groupe sulfonato, un groupe phosphino, un groupe phosphinyle, un groupe phosphono, un groupe phosphonato, un groupe amino, un groupe ammonio, ou un groupe organique ;
deux ou plus de R⁶, R⁷, R⁸, et R⁹ peuvent être liés pour former une structure cyclique et peuvent contenir une liaison hétéroatome ; et
R¹⁰ inique un groupe alkyle ayant 1 à 12 atomes de carbone,
en tant que générateur de base.

2. Utilisation d'un composé représenté par la formule (1) suivante : 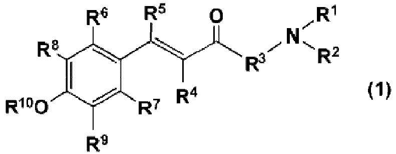
où R¹ et R² indiquent chacun indépendamment un atome d'hydrogène ou un groupe organique,
à condition qu'au moins l'un de R¹ et R² indique un groupe organique ;
R¹ et R² peuvent être liés pour former une structure cyclique et peuvent contenir une liaison hétéroatome ;
R³ indique une simple liaison ou un groupe organique ;
R⁴ et R⁵ indiquent chacun indépendamment un atome d'hydrogène, un atome d'halogène, un groupe hydroxyle, un groupe mercapto, un groupe sulfure, un groupe silyle, un groupe silanol, un groupe nitro, un groupe nitroso, un groupe sulfino, un groupe sulfo, un groupe sulfonato, un groupe phosphino, un groupe phosphinyle, un groupe phosphono, un groupe phosphonato, ou un groupe organique ;
R⁶, R⁷, R⁸, et R⁹ indiquent chacun indépendamment un atome d'hydrogène, un atome d'halogène, un groupe mercapto, un groupe sulfure, un groupe silyle, un groupe silanol, un groupe nitro, un groupe nitroso, un groupe sulfino, un groupe sulfo, un groupe sulfonato, un groupe phosphino, un groupe phosphinyle, un groupe phosphono, un groupe phosphonato, un groupe amino, un groupe ammonio, ou un groupe organique,
deux ou plus de R⁶, R⁷, R⁸, et R⁹ peuvent être liés pour former une structure cyclique et peuvent contenir une liaison hétéroatome ; et
R¹⁰ indique un atome d'hydrogène, ou un groupe alkyle ayant 1 à 12 atomes de carbone,
en tant qu'activateur d'adhérence.
